(19) Europäisches Patentamt

European Patent Office

Office européen des brevets

(11) EP 0 485 377 B1

## (12) EUROPEAN PATENT SPECIFICATION

(45) Date of publication and mention
of the grant of the patent:
**06.05.1999 Bulletin 1999/18**

(21) Application number: **90900719.7**

(22) Date of filing: **12.12.1989**

(51) Int Cl.$^6$: **G01N 33/543**, G01N 33/569, G01N 33/80

(86) International application number:
**PCT/AU89/00530**

(87) International publication number:
**WO 90/07114 (28.06.1990 Gazette 1990/15)**

(54) **SOLID PHASE IMMUNO-ASSAY WITH LABELLED CONJUGATE**

FESTPHASE-IMMUNO-TESTVERFAHREN MIT ETIKETTIERTEN KONJUGATEN

IMMUNO-ANALYSE EN PHASE SOLIDE AVEC CONJUGUE MARQUE

(84) Designated Contracting States:
**AT BE CH DE ES FR GB IT LI LU NL SE**

(30) Priority: **12.12.1988 AU 1917/88**

(43) Date of publication of application:
**20.05.1992 Bulletin 1992/21**

(73) Proprietor: **CSL LIMITED**
**Parkville, VIC 3052 (AU)**

(72) Inventor: **COX, John, Cooper**
**Bullengarook, VIC 3437 (AU)**

(74) Representative: **Allard, Susan Joyce et al**
**BOULT WADE TENNANT,**
**27 Furnival Street**
**London EC4A 1PQ (GB)**

(56) References cited:
EP-A- 0 213 653          EP-A- 0 231 830
EP-A- 0 277 723          WO-A-82/02661
AU-A- 6 981 181          US-A- 4 017 597
US-A- 4 678 747

- D.P. STITES. et al; Basic & Clinical Immunology, 4th Edition, published 1982 by Lange Medical Publications (California USA) pages 352-355.
- Clinical Chemistry, Volume 34, No. 9 issued 1988 May, J. FOLAN, et al, "Solid-Phase Enzymoimmunoassay of Estrone in Serum" pages 1843-1846.
- Clinical Chemistry, Volume 31, No. 9 issued 1985 April, G.E. VALKIRS and R. BARTON, "Immunoconcentration - A New Format for Solid-Phase Immunoassays" pages 1427-1431.
- Journal of Virological Methods, Volume 16, issued 1987 (Elsevier Scientific Publishing Company, Netherlands) I.K. MUSHAHWAR "A biotin/avidin solid-phase sandwich enzyme immunoassay for the antibody to hepatitis B surface antigen (anti-HBs)" page 11.
- A.J.C.P. Volume 90, No. 5, issued 1988 November (Bethesda Maryland, USA) R.M. BISWAS et al, "A Solid-Phase Enzyme immunoassay (ELISA) for the Detection and Quantitation of Anticardiolipin Antibody" pages 591-596.
- J. Immunol. Methods 146, 1992, p. 213 - 218

## EP 0 485 377 B1

**Description**

This invention relates to an improved immunoassay which detects the presence of and distinguishes between different analytes in a specimen.

Immunoassay kits, such as enzyme immunoassay kits, are widely used in diagnostic and research laboratories for detection and quantitation of, for example, antigens or antibodies, and usually include a labelled conjugate as a separate reagent of the kit. The present invention relates to a novel presentation of immunoassay kits whereby the conjugate is present in lyophilised form with the capture antibody or antigen.

US-A-4017597 describes a unitized solid phase radioimmunoassay kit wherein the labelled component of a binding pair, i.e. antibody or antigen, is bound to a surface. Prior to the sample to be assayed being added the radio-labelled component or conjugate is also present on the solid surface in solid phase but unbound. This is achieved by coating the bound component from solution on to the solid surface, applying a solution of the labelled component and then lyophilising the solution to leave the radio-labelled component in dried form.

The present invention relates, typically to a sandwich-type enzyme immunoassay, and as a simple example of kits using this immunoassay system, reference is made to detection of parvovirus in a canine faecal specimen. In their simplest form, available "ready-to-use" kits for this purpose would typically comprise the following separate reagents:

a. a tube or well to which is absorbed or coupled by chemical means a capture antibody preparation specific to canine parvovirus;
b. a diluent solution for resuspending the faecal specimen;
c. a conjugate solution diluted ready to use, where the conjugate is an enzyme-labelled antibody specific to canine parvovirus; and
d. an enzyme substrate solution.

Using these reagents, the assay may be performed either as a sequential or a simultaneous assay. In the former, incubation of the capture antibody with the faecal specimen and a subsequent brief wash precedes the conjugate incubation step. In the latter, these two reactions occur as a single incubation step. In this example, because of the multivalent nature of a whole virus particle, a simultaneous assay can be expected to be at least as sensitive as a sequential assay and significantly quicker and easier to perform and such expectation can be proven experimentally. Many similar examples are in the scientific literature.

The present invention simplifies the presentation and performance of such kits by removing the conjugate solution as a separate reagent and incorporating it in lyophilised form in association with the tube or well of a microtitre strip or tray. This allows test complexity to be increased, i.e. detection of different analytes within a sample but with a quicker and simpler format.

In accordance with a first aspect of the present invention there is provided an immunoassay kit for the detection of the presence in a sample of a plurality of analytes each of which comprises a first member of a specific binding pair comprising:

a. a plurality of solid substrates, each of which comprises an internal surface of a respective one of a plurality of wells in a microtitre strip or tray and each of said substrates having the other member of said specific binding pair absorbed or coupled thereto, and having a conjugate in lyophilised form associated therewith in such a manner that upon addition of an aqueous solution of said sample, the lyophilised conjugate is reconstituted and freed into the aqueous solution, and each said conjugate comprising a binding member capable of detecting the presence of said first member bound to the other member of said specific binding pair on said substrate and having a label conjugated thereto; and

b. means for detecting said label.

It will be appreciated that in the immunoassay kit broadly described above, the means for detecting the label indicates the presence of the conjugate bound to the solid substrate which, in turn, indicates the presence of the first member of said specific binding pair in said sample.

The present invention also extends to an immunoassay method for detection of the presence in a sample of a plurality of analytes each of which comprises a first member of a specific binding pair which comprises the steps of:

a. contacting said sample with a plurality of solid substrates, each of which comprises an internal surface of a respective one of a plurality of wells in a microtitre strip or tray and each of said substrates having the other member of said specific binding pair absorbed or coupled thereto and having a conjugate in lyophilised form associated therewith in such a manner that upon addition of an aqueous solution of said sample, the lyophilised conjugate is reconstituted and freed into the aqueous solution, and each said conjugate comprising a binding member capable of detecting the presence of said first member bound to the other member of said specific binding pair on said substrate and having a label conjugated thereto; and

b. detecting the binding of said conjugates to said solid substrates to indicate the presence of one or more of said first members of the specific binding pairs in said sample.

Whilst the present invention has particular application in the detection of the presence of a member of an antigen-antibody binding pair or a hapten-antibody binding pair, the invention also has application in respect of other known ligand/anti-ligand binding pairs, including ligand-receptor pairs where the ligand is a protein, steroid hormone, drug or other medicament, or the like.

Similarly, whilst specific reference is made herein to a sandwich-type enzyme immunoassay, it will be appreciated that in its broadest aspect the invention is not restricted to the use of enzyme labels, and other types of labels which are well known in immunoassays, including fluorochromes, radioisotopes and heavy metals, may also be used. Similarly, other well known types of assay formats may be used as well as the sandwich-type format, including the indirect and competitive immunoassay formats.

A particularly preferred embodiment of the invention is an immunoassay kit and method for detection of blood group antigens or antibodies in a sample.

A kit suitable for detecting blood group antigens comprises a plurality of wells in a microtitre strip or tray each of which has an antibody specific for a different blood group antigen absorbed or coupled to an internal surface thereof. The conjugate in lyophilised form is associated therewith so that upon addition of an aqueous solution of the sample the conjugate is reconstituted and freed into the aqueous solution. Conjugate comprises the same or another antibody specific for the blood group antigen and a label, the kit also including means for detection of said label.

In the case of a kit suitable for detecting blood group antibodies the plurality of wells each have coupled to the internal surface thereof an antigen, hapten or ligand specific for a different blood group. The lyophilised conjugate incorporates the same or another antigen, hapten or ligand specific for said blood group antibody or a second antibody specific for said blood group antibody or for competing therewith for binding to the first-mentioned antigen, hapten or ligand, the conjugate also including a label for detection.

This aspect of the invention also extends to corresponding immunoassay methods for the detection of blood group antigens and/or antibodies in a sample.

The essential aspect of the present invention resides in the use of the labelled conjugate in lyophilised form associated with the solid substrate. Depending on which member of the specific binding pair is absorbed or coupled to the solid substrate, the labelled conjugate may comprise an appropriate label attached to an antigen or hapten, or to an antibody, as the binding member capable of detecting the presence of the first member bound to the other member of the specific binding pair on the substrate.

The solid substrate comprises a tube or a well in an EIA or microtitre strip or tray and the bound member of the specific binding pair is absorbed or coupled to the internal surface of the tube, well or other container. Where the surface of such a tube, well or similar container is the solid substrate, the lyophilised, labelled conjugate is formed therein and remains in close association with the container in which it was dried even though it is not chemically or physically bound in any way to the substrate. On addition of water or an aqueous solution, the bound member of the specific binding pair remains bound to the substrate but the lyophilised, labelled conjugate is reconstituted and passes into solution so that it is then able to react in the immunoassay in the same way as a separately-added conjugate solution. The incorporation of the conjugate in lyophilised form associated with the solid substrate offers a number of substantial advantages in the resulting kit, including:

1. Decreased production cost. The kit will contain one less reagent container, and as a result, one less bottle and label will be required, one dispensing run with subsequent sterility test will be saved and the overall kit size and weight can be reduced.

2. Increased shelf life. Lyophilisation is the best available procedure for retaining biological activity of reagents, particularly enzyme labelled antibody, upon long term storage. At present there are three available options for storage of enzyme labelled antibody.

(i) Lyophilised in a separate container. This gives a good shelf life until first usage when the entire stock of conjugate has to be reconstituted. Stability is then that of diluted conjugate. Additionally, there are the costs of conjugate dispensing and lyophilisation and the need to supply reconstituting fluid to the user, and there is the added inconvenience of having to reconstitute conjugate before use. This is particularly inconvenient in a kit designed for field or irregular use.

(ii) As a concentrate (usually 100X in 50% glycerol). This gives reasonably good shelf life but requires that conjugate be diluted for use before each test. Availability of accurate microdilutors is essential.

(iii) Diluted ready for use. This presentation is least stable but most convenient to the user.

Since lyophilisation is a well-known technique, the preparation of the solid substrate having the lyophilised conjugate associated therewith may be carried out using known techniques and apparatus. Where the solid substrate is in the form of the well of a microtitre tray, the conjugate solution is simply added to the pre-treated well and then lyophilised, followed by sealing of the well to exclude moisture and air. Use of lyophilised conjugate in accordance with the present invention provides the shelf life of lyophilised conjugate, is cheaper to produce than any current alternative and is easier to use than any other option. Additionally, use of portion of the kit does not reduce the stability of the remainder. Thus, even in a simple assay kit the present invention confers the multiple advantages of decreased production cost and increased shelf life for manufacturer, and for the user, a quicker and simpler test format.

The advantages of this invention increase as the test complexity increases. Several examples are given below of tests which are designed to detect and distinguish between different analytes in a specimen. In these examples, in addition to the advantages cited above, the test can only be formatted in a simple and cost-effective way by utilising the improvement of the present invention.

## A. Detection and identification of snake venom in a clinical specimen.

Currently in Australia, clinical snake envenomation usually results from the bite from 5 major species, viz. tiger snake, brown snake, death adder, king brown and taipan. Rapid identification of the species can permit treatment with monovalent rather than polyvalent antivenom with a concomitant vastly reduced injection of foreign horse protein. The current diagnostic kit used to identify snake species uses affinity-absorbed antibodies to the above 5 snakes in five separate capillary tubes as the antigen capture phase and a carefully adjusted mixture of enzyme-labelled antibodies to each of the five venoms as label. The resulting test has been of considerable use but has sometimes given misleading and incorrect results with venoms from less common snakes. Extensive investigation of these false positive reactions has shown that if unabsorbed antibodies were used instead in the capture phase and followed by the homologous labelled antivenom (not a mixture) the correct antivenom for treatment would always be selected. However, to format such a kit would have required that five separate conjugate preparations be supplied, each to be added to its homologous incubation well. To perform such an assay under pressure in the field would have resulted in frequent errors. Utilising the present invention, such a test can be formatted so as to avoid these problems by lyophilising the homologous conjugate within the appropriate well. The test can then be performed under field conditions with minimal chance of error, or false diagnosis and in much less time than the current test.

## B. Detection of non-bacterial pathogens in human faeces.

Within Australia and indeed in most developed countries of the world, there are four major non-bacterial pathogens which cause diarrhoea in adults and children. These are the two protozoans <u>Giardia</u> and <u>Cryptosporidia</u> and the two viruses rotavirus and enteric adenovirus. The two protozoa can exist either as cysts or as free-growing trophozoites. These two forms have a varying array of surface antigens, few if any of which have been characterised. Specific identification can be achieved most reliably by use of a good polyclonal antiserum, production of which does not require detailed knowledge of antigenic structure and which antigens are immunodominant. Conversely, viruses are antigenically far more simple and, in fact, specific identification of an enteric adenovirus from other closely related viruses is best achieved by use of a monoclonal antiserum specific for a single epitope. Thus, to identify these four enteric pathogens in a single diagnostic kit would necessitate four separate conjugate preparations or otherwise two separate preparations (one polyclonal, one monoclonal) each at double strength. This is because polyclonal antiserum from any species naturally will contain antibodies to the immunoglobulins of other species. Thus, for example, polyclonal rabbit serum can be expected to react with and precipitate mouse immunoglobulins etc. It is possible that such reactivity could be removed by affinity absorption permitting a single conjugate mix to be produced. However additional to the cost of this extra process the final conjugate mixture would need to be at 4X strength. The present invention again overcomes all these problems because the homologous capture and label antibodies are presented in the incubation well ready for use.

## C. Detection of meat species and meat contamination.

Irregularities in the meat industry has led, at various times, to meat from one species being substituted for that of another species. For religious, public health and other reasons, it is desirable to be able to detect such substitutions. For this purpose, a kit has been developed to identify bovine, buffalo, camel, donkey/horse, goat, kangaroo, pig and sheep. For reasons of economy, polyclonal antisera were raised in rabbits (for all species) and sheep (for all but anti-sheep where goats were used). In all cases, rabbits produced sera of lesser quality, and in some cases, following absorption to render them species specific, their sensitivity was significantly less than the required 1%. However, although the sheep anti-goat was of high specificity and sensitivity, the goat anti-sheep was unsatisfactory and monoclonals to sheep were developed instead. Using these reagents, it was straightforward to produce a kit to examine large numbers of meat samples for the presence of any single species, but a preference was expressed in the market place for a test which would check for all eight species in a single kit. Once again, this was only possible by use of eight separate conjugates which had to be added to the eight appropriate wells after the primary incubation (otherwise the mouse anti-sheep would have precipitated the sheep-derived immunoglobulins). The present invention permits a single incubation assay for multi-species testing even though antisera could not be presented as single-mix conjugate.

## D. Solid phase blood grouping.

Traditionally, determination of human blood groups has required forward and reverse matchings using suspensions of washed cells and plasma respectively. Presence of antigen or antibody is judged by eye as presence of agglutination. Various ways to automate these procedures and reduce subj-ectivity have been devised. However, all these attempts have been characterised by a number of shortcomings including the need to separate cells from plasma, expensive equipment and a continuing need to detect anti-red cell autoantibodies in a separate test. By use of the present invention, it is possible to separately detect antigens A, B, D, and H and antibodies to A, B, H and self red cells in a rapid, non-subjective, fully automatable test using existing equipment. In the absence of this new approach, the test would require two incubations with different conjugates to be added to different wells.

Further features of the present invention will be apparent from the following Examples, which are included by way of exemplification and not limitation of the invention.

## EXAMPLE 1

### Snake Venom Detection Kit

(a) Preparation of Antisera.
High-titre antisera to snake venom is prepared by hyperimmunization of rabbits with increasing amounts of venom in Freund's adjuvant. The primary immunization course involves 10 weekly doses deep intramuscularly; booster doses are subcutaneous. Antisera are raised to each of the snake venoms of importance, viz: tiger (TSV), brown (BSV), king brown, taipan and death adder.
(b) Processing of antisera.

(i) Pure IgG is prepared by Protein A affinity purification according to established methods for absorption and elution of rabbit IgG. The material is checked for purity by polyacrylamide gel electrophoresis (PAGE). A single band of MW 150 kD should be present.
(ii) Purified IgG is enzymically degraded to $Fab_2$ by controlled reaction with solid-phase pepsin. The reaction is terminated by removal of the solid phase. The material is checked for purity by PAGE. A single band of MW 100kD should be present.
(iii) Purified IgG is chemically coupled to the enzyme horse radish peroxidase (HRP) by the periodate procedure of Nakane and Kawaoi (1974), J.Histochem. Cytochem. 22, 1084.

(c) Description of kit reagents.
An assay kit for field use comprises the following reagents:

(i) an 8 well strip
(ii) sample diluent buffer
(iii) enzyme substrate buffer ($H_2O_2$ solution stabilized) 2x strength
(iv) chromogen solution 2x strength.

(d) Preparation of 8 well strip.
The contents of the eight wells are summarized below:

Well 1    Coating antibody: Rabbit anti-TSV
          Conjugate: Rabbit anti-TSV-HRP labelled
Well 2    Coating antibody: Rabbit anti-BSV
          Conjugate: Rabbit anti-BSV-HRP labelled
Well 3    Coating antibody: Rabbit anti-Taipan
          Conjugate: Rabbit anti-Taipan-HRP labelled
Well 4    Coating antibody: Rabbit anti-Death Adder
          Conjugate: Rabbit anti-Death Adder-HRP labelled
Well 5    Coating antibody: Rabbit anti-King Brown
          Conjugate: Rabbit anti-King Brown-HRP labelled
Well 6    Coating antibody: Normal rabbit IgG
          Conjugate: Rabbit anti-TSV-HRP labelled
Well 7    Coating antibody: Normal rabbit IgG
          Conjugate: Sheep anti-rabbit IgG-HRP labelled
Well 8    Blank - orientation well.

The 8 well strips are prepared according to the following steps:

1. Coat: 10μg/mL rabbit IgG in 0.05 M sodium bicarbonate buffer, pH 9.5. Add 100 μL/well and incubate overnight at 4°C then flick out.
2. Postcoat: 1 mg/mL casein, 5% lactose in PBS pH 7.2. Add 150 μL/well and incubate 1 hr at room temperature, then flick out.
3. Stabilization: 5% glucosamine in distilled water. Add 150 μl/well and incubate 5-10 minutes at room temperature. Flick out.
4. Conjugate: Add 5 μl conjugate at 12.5x optimal level (estimated when used wet, in a simultaneous assay - 50 μL sample, 50 μl conjugate). Conjugate concentrate diluted in the following diluent buffer - 1 μg/mL casein, 5% lactose in sorbitol buffer.
5. Lyophilization: Plates are immediately placed on shelves in a freeze dryer, and the shelf temperature dropped to -40°C. Lyophilization takes place under vacuum. Following lyophilization, 8 well strips are individually sealed to exclude moisture and air.

(e) Typical results for laboratory tests.

Results for 10 ng/mL venoms are shown in Table 1. It can be seen that the strongest signal is always given by the homologous venom; cross reacting signals being around one quarter.

(f) Examination of clinical specimens.

The kit has been designed for use with serum, urine and swab from a bite site. Although primarily intended for clinical use, it may also be used for various veterinary applications, especially for companion animals. The use of serum from whatever source can frequently lead to high, non-specific signals due to anti-species immunoglobulins (e.g. rheumatoid factor). Such non-specific reactivity can be effectively removed by use of one or more of:

(i) Fab'$_2$ rather than whole IgG as capture antibody.
(ii) Fab'$_2$ rather than whole IgG for conjugation to HRP.
(iii) Blocking IgG in the incubation buffer.

Such procedures have been used to completely remove non-specific reactivity from this test.

(g) Assay sensitivity.

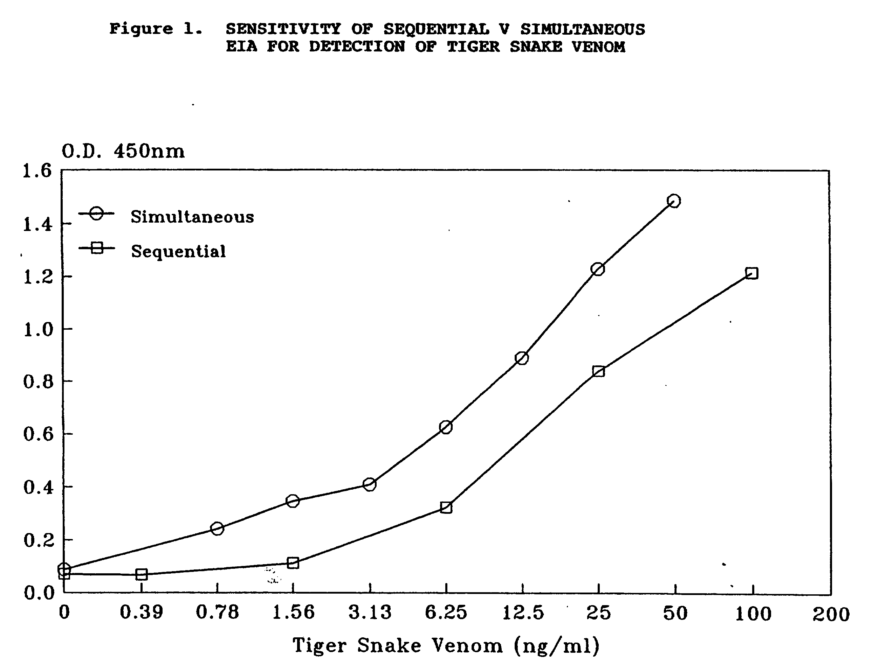

Sensitivity was determined for the sequential and simultaneous assay systems. The results of a typical assay for tiger snake venom is shown in Figure 1. It can be seen that the simultaneous assay, which forms the basis for this assay format is actually more sensitive using a single 10 minute incubation than is the sequential assay with 10 minutes for each incubation stage. Sensitivity was less than 1 μg/mL (read as blue signal when controls were colourless).

(h) Kit Stability.

To determine kit stability, kits were stored at 37°C and 4°C and tested in parallel using three levels of venom. Stability was calculated as the OD for each venom level at 37°C as a percentage of that at 4°C

$$\text{i.e. } \% \text{ drop} = \frac{\text{OD } 4°C - \text{OD } 37°C}{\text{OD } 4°C} \times 100$$

Tests were performed at doubling time intervals from week 1 and plotted to calculate a projected half life. The product half life at 37°C was estimated at 40 weeks.

TABLE 1

| O.D. 450nm obtained for venoms at 10ng/ml | | | | | | |
|---|---|---|---|---|---|---|
| | | Ti | Br | Tp | DA | KB |
| Identification of Well | Ti | 1.267 | 0.131 | 0.365 | 0.274 | 0.464 |
| | Br | 0.101 | 0.528 | 0.074 | 0.092 | 0.078 |
| | Tp | 0.037 | 0.042 | 0.957 | 0.088 | 0.065 |
| | DA | 0.308 | 0.087 | 0.215 | 0.726 | 0.238 |
| | KB | 0.156 | 0.125 | 0.143 | 0.141 | 1.340 |
| | - | 0.227 | 0.075 | 0.164 | 0.083 | 0.070 |
| | + | 1.160 | 1.525 | 1.076 | 1.154 | 1.114 |
| (Ti = tiger; Br = brown; Tp = taipan; DA = death adder; KB = king brown.) | | | | | | |

## EXAMPLE 2

### Solid phase blood grouping kit.

The kit described is a simple 8 well kit for forward and reverse grouping and direct Coombs.

(a) Preparation of reagents.
Antisera to selected blood group antigens are most conveniently sourced as monoclonal antibodies (MAbs) with defined specificity and affinity. However, polyclonal antisera can be used. The following antisera are required:

(i) MAb-anti A - must have specificity to all A sub-groups.
(ii) MAb-anti B
(iii) MAb-anti H
(iv) MAb-anti D
(v) anti-human immunoglobulin (rabbit, sheep or mouse).

(b) Processing of selected antisera.

(i) Pure immunoglobulin is prepared by ion exchange or affinity purification as appropriate according to established methods. The resulting material is checked for purity by PAGE. A single band should be present.
(ii) Purified immunoglobulin is chemically coupled to the enzyme horse radish peroxidase (HRP) by the periodate procedure of Nakane and Kawaoi (1974), J.Histochem. Cytochem, 22, 1084.

(c) Other reagents.
Synthetic glycoproteins (Biocarb Chemicals, Lund, Sweden) in which the oligosaccharide is specific to blood group antigens and is coupled via a spacer to a protein, e.g. casein.
(d) Description of kit reagents.

(i) a 96 well EIA plate containing 12x8 well strips
(ii) diluent buffer for whole blood
(iii) wash buffer 20x strength
(iv) enzyme substrate buffer 2x strength
(v) chromogen solution 2x strength
(vi) stop solution.

(e) Format of an 8 well strip.

Well 1    Coating antibody: MAb-anti-A

Conjugate: MAb-anti-H-HRP
Well 2    Coating antibody: MAb-anti-B
Conjugate: MAb-anti-H-HRP
Well 3    Coating antibody: MAb-anti-H
Conjugate: MAb-anti-H-HRP
Well 4    Coating antibody: MAb-anti-D
Conjugate: MAb-anti-H-HRP
Well 5    Coating antigen: A-trisaccharide-casein
Conjugate: A-trisaccharide-casein-HRP
Well 6    Coating antigen: B-trisaccharide-casein
Conjugate: B-trisaccharide-casein-HRP
Well 7    Coating antigen: 2'-fucosyllactosamine-casein
Conjugate: 2-fucosyllactosamine-casein-HRP
Well 8    Coating antibody: MAb-anti-H
Conjugate: antihuman immunoglobulin-HRP

In the above format, well 3 will act as a positive control (rare exception Bombay group) and well 7 as a negative control (rare exception Bombay group).

In one alternative configuration, the conjugate in wells 5, 6 and 7 will be, respectively MAb-anti-A-HRP, MAb-Anti-B-HRP and MAb-anti-H-HRP. The 8 well strips are prepared as described in Example 2. Monoclonal antibodies were coated at 1 $\mu$g/mL, synthetic glycoproteins at 5-10 pg/mL.

(f) Typical test results.

Whole blood is diluted 1 in 25 to 1 in 50 in diluent buffer then 100 $\mu$L added to each well of a strip, mixed then allowed to stand for 5 minutes. Wells are then washed thoroughly and substrate/chromogen added. In a typical set of results, readings as presented in Table 2 were obtained.

TABLE 2

| Typical Test Results | | | | | | | |
|---|---|---|---|---|---|---|---|
| **BLOOD GROUP** | | | | | | | |
| | O Pos | $A_1$ Pos | $A_2$ Neg | $A_2$B Neg | B Pos | Neg | Coombs Pos. $A_1$ Pos |
| Well 1 | .073 | 1.426 | 1.1281 | 1.200 | 0.084 | 0.057 | 1.523 |
| 2 | .102 | 0.083 | 0.077 | 1.277 | 1.520 | 0.064 | 0.071 |
| 3 | 1.584 | 2.230 | 2.663 | 2.675 | 2.682 | 2.737 | 2.131 |
| 4 | 1.176 | 2.286 | 0.127 | 2.371 | 1.706 | 0.053 | 1.982 |
| 5 | 1.391 | 0.151 | 0.168 | 0.251 | 1.108 | 1.659 | 0.120 |
| 6 | 0.976 | 1.112 | 1.380 | 0.126 | 0.098 | 1.504 | 1.365 |
| 7 | 0.117 | 0.079 | 0.055 | 0.067 | 0.103 | 0.041 | 0.080 |
| 8 | 0.051 | 0.062 | 0.041 | 0.040 | 0.055 | 0.053 | 1.720 |
| * OD - 450 nm | | | | | | | |

## EXAMPLE 3

### Solid phase blood group antibody screen.

This antibody screen can be included as part of the 8 well test described in Example 2 above.

Two panels of group O red cells are prepared by careful selection. Between them, these panels must contain all of the following antigens: C, c, D, E, e, M, N, S, s, $P_1$, K, k, $Fy^a$, $Fy^b$, $Jk^a$, $Jk^b$, $Le^a$ and $Le^b$.

As described previously, these cells would function as capture antigen either by direct attachment of cells to the walls of the 96 well plates or indirectly via MAb-anti-H capture. The conjugate-antihuman immunoglobulin-HRP, is lyophilized as described previously.

## Claims

1. An immunoassay kit for the detection of the presence in a sample of a plurality of analytes each of which comprises a first member of a specific binding pair, comprising:

   a. a plurality of solid substrates each of which comprises an internal surface of a respective one of a plurality of wells in a microtitre strip or tray and each of said substrates having the other member of a said specific binding pair absorbed or coupled thereto, and a conjugate in lyophilised form associated therewith in such a manner that upon addition of an aqueous solution of said sample, the lyophilised conjugate is reconstituted and freed into the aqueous solution, and each said conjugate comprising a binding member capable of detecting the presence of said first member bound to the other member of said specific binding pair on said substrate and having a label conjugated thereto; and

   b. means for detecting said label.

2. A kit according to claim 1, wherein said wells are sealed to exclude moisture and air.

3. A kit according to any preceding claim wherein each said conjugate comprises an enzyme label.

4. A kit according to claim 3 wherein said enzyme is horseradish peroxidase.

5. A kit according to any of claims 1 to 4, for detection of a plurality of antigens in a sample, wherein each of said solid substrates has an antibody specific for a said antigen absorbed or coupled thereto, and each said conjugate comprises the same or another antibody specific for said antigen having a label conjugated thereto.

6. A kit according to claim 5, wherein said antigens are bacterial, viral or protozoal antigens.

7. A kit according to claim 6, wherein said antigens include canine parvovirus.

8. A kit according to claim 5, wherein said antigens are snake venoms.

9. A kit according to claim 5, wherein said antigens are blood group antigens.

10. A kit according to any of claims 1 to 4 for detection of a plurality of antibodies in a sample, wherein each of said solid substrates has an antigen, hapten or other ligand specific for a said antibody absorbed or coupled thereto, and each said conjugate comprises the same or another antigen, hapten or ligand specific for said antibody, or a second antibody specific for said first-mentioned antibody or competing with said first-mentioned antibody for binding to said first-mentioned antigen, hapten or ligand, having a label conjugated thereto.

11. A kit according to claim 10, wherein said antibodies are blood group antibodies.

12. A kit according to claim 9 which comprises a plurality of wells, each of said wells having an antibody specific for a different blood group antigen absorbed or coupled to an internal surface thereof, and each said lyophilised conjugate comprising the same or another antibody specific for said blood group antigen having a label conjugated thereto.

13. A kit according to claim 12, wherein said plurality of wells comprises wells having anti-A, anti-B, anti-H and/or anti-D antibodies absorbed or coupled thereto.

14. A kit according to claim 11 which comprises a plurality of wells, each of said wells having an antigen, hapten or ligand specific for a different blood group antibody absorbed or coupled to an internal surface thereof, and each said lyophilised conjugate comprising the same or another antigen, hapten or ligand specific for said blood group antibody or a second antibody specific for said blood group antibody or competing with said blood group antibody for binding to said first-mentioned antigen, hapten or ligand, having a label conjugated thereto.

15. A kit according to claim 14, wherein said plurality of wells includes one or more wells having synthetic glycoproteins absorbed or coupled thereto, each of said synthetic glycoproteins comprising an oligosaccharide binding to a blood group antibody coupled to a protein.

**16.** An immunoassay method for detection of the presence in a sample of a plurality of analytes each of which comprises a first member of a specific binding pair which comprises the steps of:

a. contacting said sample with a plurality of solid substrates each of which comprises an internal surface of a respective one of a plurality of wells in a microtitre strip or tray and each of said substrates having the other member of a said specific binding pair absorbed or coupled thereto, and a conjugate in lyophilised form associated therewith in such a manner that upon addition of an aqueous solution of said sample, the lyophilised conjugate is reconstituted and freed into the aqueous solution, and each said conjugate comprising a binding member capable of detecting the presence of said first member bound to the other member of said specific binding pair on said substrate and having a label conjugated thereto; and

b. detecting the binding of said conjugates to said solid substrates to indicate the presence of one or more of said first members of the specific binding pairs in said sample.

**17.** A method according to claim 16, wherein each said conjugate comprises an enzyme label.

**18.** A method according to claim 17, wherein said enzyme is horseradish peroxidase.

**19.** A method according to any of claims 16 to 18, for detection of a plurality of antigens in a sample, wherein each of said solid substrates has an antibody specific for a said antigen absorbed or coupled thereto, and each said conjugate comprises the same or another antibody specific for said antigen having a label conjugated thereto.

**20.** A method according to claim 19, wherein said antigens are bacterial, viral or protozoal antigens.

**21.** A method according to claim 20, wherein said antigens include canine parvovirus.

**22.** A method according to claim 19, wherein said antigens are snake venoms.

**23.** A method according to claim 19, wherein said antigens are blood group antigens.

**24.** A method according to any of claims 16 to 18 for detection of a plurality of antibodies in a sample, wherein each of said solid substrates has an antigen, hapten or other ligand specific for a said antibody absorbed or coupled thereto, and each said conjugate comprises the same or another antigen, hapten or ligand specific for said antibody, or a second antibody specific for said first-mentioned antibody or competing with said first-mentioned antibody for binding to said first-mentioned antigen, hapten or ligand, having a label conjugated thereto.

**25.** A method according to claim 24, wherein said antibodies are blood group antibodies.

**26.** A method according to claim 23 which comprises contacting said sample with a plurality of wells, each of said wells having an antibody specific for a different blood group antigen absorbed or coupled to an internal surface thereof, each said lyophilised conjugate comprising the same or another antibody specific for said blood group antigen having a label conjugated thereto and detecting binding of said conjugates to said well surfaces to indicate the presence of one or more of said blood group antigens in said sample.

**27.** A method according to claim 26, wherein said plurality of wells comprises wells having anti-A, anti-B, anti-H and/or anti-D antibodies absorbed or coupled thereto.

**28.** A method according to claim 25 which comprises contacting said sample with plurality of wells, each of said wells having an antigen, hapten or ligand specific for a different blood group antibody absorbed or coupled to an internal surface thereof each said lyophilised conjugate comprising the same or another antigen, hapten or ligand specific for said blood group antibody, or a second antibody specific for said blood group antibody or competing with said blood group antibody for binding to said first-mentioned antigen, hapten or ligand, having a label conjugated thereto and detecting binding of said conjugates to said well surfaces to indicate the presence of one or more of said blood group antibodies in said sample.

**29.** A method according to claim 28, wherein said plurality of wells includes one or more wells having synthetic glycoproteins absorbed or coupled thereto, each of said synthetic glycoproteins comprising an oligosaccharide binding to a blood group antibody coupled to a protein.

**30.** A kit according to claim 1, wherein each said member of said specific binding pair absorbed or coupled to a solid substrate is stabilised with glucosamine prior to lyophilisation of said conjugate.

**31.** A kit according to claim 12, wherein each said antibody absorbed or coupled to the internal surface of a well is stabilised with glucosamine prior to lyophilisation of said conjugate.

**32.** A kit according to claim 14, wherein each said antigen, hapten or ligand absorbed or coupled to the internal surface of a well is stabilised with glucosamine prior to lyophilisation of said conjugate.

**33.** A method according to claim 16, wherein each said member of said specific binding pair absorbed or coupled to a solid substrate is stabilised with glucosamine prior to lyophilisation of said conjugate.

**34.** A method according to claim 26, wherein each said antibody absorbed or coupled to the internal surface of a well is stabilised with glucosamine prior to lyophilisation of said conjugate.

**35.** A method according to claim 28, wherein each said antigen, hapten or ligand absorbed or coupled to the internal surface of a well is stabilised with glucosamine prior to lyophilisation of said conjugate.

**Patentansprüche**

**1.** Immunoassaykit zum Nachweis des Vorhandenseins einer Mehrzahl von Analyten in einer Probe, von denen jeder ein erstes Element eines spezifischen Bindungspaares enthält, umfassend:

a. eine Mehrzahl fester Substrate, von denen jedes eine innere Oberfläche einer jeweiligen (Ausnehmung) einer Mehrzahl von Ausnehmungen in einem Mikrotiterstreifen oder einer Mikrotiterplatte umfaßt und jedes der Substrate das andere Element des genannten spezifischen Bindungspaares daran absorbiert oder gekuppelt aufweist und ein Konjugat in lyophilisierter Form dergestalt damit assoziiert enthält, daß bei Zugabe einer wäßrigen Lösung der Probe das lyophilisierte Konjugat wiederhergestellt und in die wäßrige Lösung freigesetzt wird, wobei das jeweilige Konjugat ein Bindungselement mit der Fähigkeit zum Nachweis des Vorhandenseins des ersten Elements, das an das andere Element des spezifischen Bindungspaares auf dem Substrat gebunden ist, umfaßt und eine daran konjugierte Markierung aufweist, und
b. ein Mittel zum Nachweis der Markierung.

**2.** Kit nach Anspruch 1, wobei die Ausnehmungen zum Ausschluß von Feuchtigkeit und Luft versiegelt sind.

**3.** Kit nach einem der vorhergehenden Ansprüche, wobei jedes Konjugat eine Enzymmarkierung umfaßt.

**4.** Kit nach Anspruch 3, wobei das Enzym aus Meerrettichperoxidase besteht.

**5.** Kit nach einem der Ansprüche 1 bis 4 zum Nachweis einer Mehrzahl von Antigenen in einer Probe, wobei jedes der festen Substrate einen für das betreffende Antigen spezifischen Antikörper daran absorbiert oder gekuppelt enthält und das jeweilige Konjugat denselben oder einen anderen Antikörper mit Spezifität für das Antigen mit einer daran konjugierten Markierung umfaßt.

**6.** Kit nach Anspruch 5, wobei die Antigene aus Bakterien-, Viren- oder Protozoen-Antigenen bestehen.

**7.** Kit nach Anspruch 6, wobei die Antigene Hunde-Parvovirus umfassen.

**8.** Kit nach Anspruch 5, wobei die Antigene aus Schlangengiften bestehen.

**9.** Kit nach Anspruch 5, wobei die Antigene aus Blutgruppenantigenen bestehen.

**10.** Kit nach einem der Ansprüche 1 bis 4 zum Nachweis einer Mehrzahl von Antikörpern in einer Probe, wobei jedes der festen Substrate ein(en) Antigen, Hapten oder sonstigen Liganden mit Spezifität für den Antikörper daran absorbiert oder gekuppelt aufweist und das jeweilige Konjugat dasselbe (denselben) oder ein(en) sonstiges (sonstigen) Antigen, Hapten oder Liganden mit Spezifität für den Antikörper oder einen zweiten Antikörper, der für den erstgenannten Antikörper spezifisch ist oder mit dem erstgenannten Antikörper um eine Bindung an das (den) erstgenannte(n) Antigen, Hapten oder Liganden konkurriert, mit einer damit konjugierten Markierung umfaßt.

11. Kit nach Anspruch 10, wobei die Antikörper aus Blutgruppenantikörpern bestehen.

12. Kit nach Anspruch 9, umfassend eine Mehrzahl von Ausnehmungen, wobei jede dieser Ausnehmungen einen an ihre innere Oberfläche absorbierten oder gekuppelten Antikörper, der für ein anderes Blutgruppenantigen spezifisch ist, aufweist und das jeweilige lyophilisierte Konjugat denselben oder einen anderen Antikörper mit Spezifität für das Blutgruppenantigen mit einer daran konjugierten Markierung umfaßt.

13. Kit nach Anspruch 12, wobei die Mehrzahl von Ausnehmungen Ausnehmungen mit daran absorbierten oder gekuppelten Anti-A-, Anti-B-, Anti-H- und/oder Anti-D-Antikörpern umfaßt.

14. Kit nach Anspruch 11, umfassend eine Mehrzahl von Ausnehmungen, von denen jede ein(en) an ihre innere Oberfläche absorbiertes (absorbierten) oder gekuppeltes (gekuppelten) Antigen, Hapten oder Liganden mit Spezifität für einen anderen Blutgruppenantikörper aufweist, und wobei das jeweilige lyophilisierte Konjugat dasselbe (denselben) oder ein(en) anderes (anderen) Antigen, Hapten oder Liganden mit Spezifität für den Blutgruppenantikörper oder einen zweiten Antikörper, der für den Blutgruppenantikörper spezifisch ist oder mit dem Blutgruppenantikörper um eine Bindung an das (den) erstgenannte(n) Antigen, Hapten oder Liganden konkurriert, mit einer daran konjugierten Markierung umfaßt.

15. Kit nach Anspruch 14, wobei die Mehrzahl der Ausnehmungen eine oder mehrere Ausnehmung(en) mit daran absorbierten oder gekuppelten synthetischen Glykoproteinen enthält, wobei jedes der synthetischen Glykoproteine ein an ein Protein gekuppeltes Oligosaccharid, das an einen Blutgruppenantikörper bindet, aufweist.

16. Immunoassayverfahren zum Nachweis des Vorhandenseins einer Mehrzahl von Analyten in einer Probe, von denen jeder ein erstes Element eines spezifischen Bindungspaares umfaßt, umfassend die folgenden Stufen: a. Kontaktieren der Probe mit einer Mehrzahl fester Substrate, wobei jedes derselben eine innere Oberfläche einer jeweiligen (Ausnehmung) einer Mehrzahl von Ausnehmungen in einem Mikrotiterstreifen oder einer Mikrotiterplatte umfaßt und jedes der Substrate das andere Element des genannten spezifischen Bindungspaares daran absorbiert oder gekuppelt aufweist und ein Konjugat in lyophilisierter Form dergestalt damit assoziiert enthält, daß bei Zugabe einer wäßrigen Lösung der Probe das lyophilisierte Konjugat wiederhergestellt und in die wäßrige Lösung freigesetzt wird, wobei das jeweilige Konjugat ein Bindungselement mit der Fähigkeit zum Nachweis des Vorhandenseins des ersten Elements, das an das andere Element des spezifischen Bindungspaares auf dem Substrat gebunden ist, umfaßt und eine daran konjugierte Markierung aufweist, und b. Nachweisen der Bindung der Konjugate an die festen Substrate zur Anzeige des Vorhandenseins eines oder mehrerer der ersten Elemente der spezifischen Bindungspaare in der Probe.

17. Verfahren nach Anspruch 16, wobei das jeweilige Konjugat eine Enzymmarkierung umfaßt.

18. Verfahren nach Anspruch 17, wobei das Enzym aus Meerrettichperoxidase besteht.

19. Verfahren nach einem der Ansprüche 16 bis 18 zum Nachweis einer Mehrzahl von Antigenen in einer Probe, wobei jedes der festen Substrate einen für das betreffende Antigen spezifischen Antikörper daran absorbiert oder gekuppelt enthält und das jeweilige Konjugat denselben oder einen anderen Antikörper mit Spezifität für das Antigen mit einer daran konjugierten Markierung umfaßt.

20. Verfahren nach Anspruch 19, wobei die Antigene aus Bakterien-, Viren- oder Protozoen-Antigenen bestehen.

21. Verfahren nach Anspruch 20, wobei die Antigene Hunde-Parvovirus umfassen.

22. Verfahren nach Anspruch 19, wobei die Antigene aus Schlangengiften bestehen.

23. Verfahren nach Anspruch 19, wobei die Antigene aus Blutgruppenantigenen bestehen.

24. Verfahren nach einem der Ansprüche 16 bis 18 zum Nachweis einer Mehrzahl von Antikörpern in einer Probe, wobei jedes der festen Substrate ein(en) Antigen, Hapten oder sonstigen Liganden mit Spezifität für den Antikörper daran abosrbiert oder gekuppelt aufweist und das jeweilige Konjugat dasselbe (denselben) oder ein(en) sonstiges (sonstigen) Antigen, Hapten oder Liganden mit Spezifität für den Antikörper oder einen zweiten Antikörper, der für

EP 0 485 377 B1

den erstgenannten Antikörper spezifisch ist oder mit dem erstgenannten Antikörper um eine Bindung an das (den) erstgenannte(n) Antigen, Hapten oder Liganden konkurriert, mit einer daran konjugierten Markierung umfaßt.

25. Verfahren nach Anspruch 24, wobei die Antikörper aus Blutgruppenantikörpern bestehen.

26. Verfahren nach Anspruch 23, umfassend das Kontaktieren der Probe mit einer Mehrzahl von Ausnehmungen, von denen jede dieser Ausnehmungen einen an ihre innere Oberfläche absorbierten oder gekuppelten Antikörper, der für ein anderes Blutgruppenantigen spezifisch ist, aufweist, und wobei das jeweilige lyophilisierte Konjugat denselben oder einen anderen Antikörper mit Spezifität für das Blutgruppenantigen mit einer daran konjugierten Markierung umfaßt, und den Nachweis einer Bindung der Konjugate an die Ausnehmungsberflächen zur Anzeige des Vorhandenseins eines oder mehrerer der Blutgruppenantigene in der Probe.

27. Verfahren nach Anspruch 26, wobei die Mehrzahl von Ausnehmungen Ausnehmungen mit daran absorbierten oder gekuppelten Anti-A-, Anti-B-, Anti-H- und/oder Anti-D-Antikörpern umfaßt.

28. Verfahren nach Anspruch 25, umfassend das Kontaktieren der Probe mit einer Mehrzahl von Ausnehmungen, von denen jede ein(en) an ihre innere Oberfläche absorbiertes (absorbierten) oder gekuppeltes (gekuppelten) Antigen, Hapten oder Liganden mit Spezifität für einen anderen Blutgruppenantikörper aufweist, und wobei das jeweilige lyophilisierte Konjugat dasselbe (denselben) oder ein(en) anderes (anderen) Antigen, Hapten oder Liganden mit Spezifität für den Blutgruppenantikörper oder einen zweiten Antikörper, der für den Blutgruppenantikörper spezifisch ist oder mit dem Blutgruppenantikörper um eine Bindung andas (den) erstgenannte(n) Antigen, Hapten oder Liganden konkurriert, mit einer daran konjugierten Markierung umfaßt, und den Nachweis einer Bindung der Konjugate an die Ausnehmungsoberflächen zur Anzeige des Vorhandenseins eines oder mehrerer der Blutgruppenantikörper in der Probe.

29. Verfahren nach Anspruch 28, wobei die Mehrzahl der Ausnehmungen eine oder mehrere Ausnehmung(en) mit daran absorbierten oder gekuppelten synthetischen Glykoproteinen enthält, wobei jedes der synthetischen Glykoproteine ein an ein Protein gekuppeltes Oligosaccharid, das einen Blutgruppenantikörper bindet, aufweist.

30. Kit nach Anspruch 1, wobei jedes der an ein festes Substrat absorbierten oder gekuppelten Elemente des spezifischen Bindungspaares vor dem Lyophilisieren des Konjugats mit Glucosamin stabilisiert ist.

31. Kit nach Anspruch 12, wobei der jeweilige, an die innere Oberfläche einer Ausnehmung absorbierte oder gekuppelte Antikörper vor dem Lyophilisieren des Konjugats mit Glucosamin stabilisiert ist.

32. Kit nach Anspruch 14, wobei das (der) jeweilige, an die innere Oberfläche einer Ausnehmung absorbierte oder gekuppelte Antigen, Hapten oder Ligand vor dem Lyophilisieren des Konjugats mit Glucosamin stabilisiert ist.

33. Verfahren nach Anspruch 16, wobei jedes der an ein festes Substrat absorbierten oder gekuppelten Elemente des spezifischen Bindungspaares vor dem Lyophilisieren des Konjugats mit Glucosamin stabilisiert ist.

34. Verfahren nach Anspruch 26, wobei der jeweilige, an die innere Oberfläche einer Ausnehmung absorbierte oder gekuppelte Antikörper vor dem Lyophilisieren des Konjugats mit Glucosamin stabilisiert ist.

35. Verfahren nach Anspruch 28, wobei das (der) jeweilige, an die innere Oberfläche einer Ausnehmung absorbierte oder gekuppelte Antigen, Hapten oder Ligand vor dem Lyophilisieren des Konjugats mit Glucosamin stabilisiert ist.

**Revendications**

1. Kit d'immunoanalyse destiné à détecter la présence dans un échantillon d'une pluralité d'analytes comprenant chacun un premier membre d'un couple de liaison spécifique, comprenant:

   a. une pluralité de substrats solides comprenant chacun une surface interne de l'un respectif d'une pluralité de puits d'une bandelette ou d'une boîte de microtitration, chacun desdits substrats portant l'autre membre dudit couple de liaison spécifique absorbé sur ou couplé à celui-ci et un conjugué sous forme lyophilisée auquel il est associé de telle manière qu'après addition d'une solution aqueuse dudit échantillon, le conjugué lyophilisé soit reconstitué et libéré dans la solution aqueuse, ledit conjugué comprenant un membre de liaison susceptible de détecter la présence dudit premier membre lié à l'autre membre dudit couple de liaison spécifique sur ledit substrat et possédant un marqueur conjugué à celui-ci; et

b. un moyen de détection dudit marqueur.

2. Kit selon la revendication 1, dans lequel lesdits puits sont scellés afin d'être maintenus à l'abri de l'humidité et de l'air.

3. Kit selon l'une quelconque des revendications précédentes dans lequel chacun desdits conjugués comprend un marqueur enzymatique.

4. Kit selon la revendication 3 dans lequel ladite enzyme est la peroxydase de raifort.

5. Kit selon l'une des revendications 1 à 4 destiné à détecter une pluralité d'antigènes dans un échantillon, où chacun desdits substrats solides porte un anticorps spécifique d'un dit antigène absorbé sur ou couplé à celui-ci et chacun desdits conjugués comprend le même ou un autre anticorps spécifique dudit antigène auquel est conjugué un marqueur.

6. Kit selon la revendication 5 dans lequel lesdits antigènes sont des antigènes bactériens, viraux ou protozoaires.

7. Kit selon la revendication 6 dans lequel lesdits antigènes incluent le parvovirus canin.

8. Kit selon la revendication 5 dans lequel lesdits antigènes sont des venins de serpent.

9. Kit selon la revendication 5 dans lequel lesdits antigènes sont des antigènes de groupes sanguins.

10. Kit selon l'une des revendications 1 à 4 destiné à détecter une pluralité d'anticorps dans un échantillon, dans lequel chacun desdits substrats solides porte un antigène, un haptène ou un autre ligand spécifique d'un dit anticorps absorbé sur ou couplé à celui-ci, et chacun desdits conjugués comprend le même ou un autre antigène, haptène ou ligand spécifique dudit anticorps ou un second antigène spécifique dudit premier anticorps ou en compétition avec ledit premier anticorps vis-à-vis de la liaison audit premier antigène, haptène ou ligand, auquel est conjugué un marqueur.

11. Kit selon la revendication 10 dans lequel lesdits anticorps sont des anticorps des groupes sanguins.

12. Kit selon la revendication 9 qui comprend une pluralité de puits, chacun desdits puits contenant un anticorps spécifique d'un antigène d'un groupe sanguin différent absorbé ou couplé sur une surface interne de celui-ci, et chacun desdits conjugués lyophilisés comprenant le même ou un autre anticorps spécifique dudit antigène d'un groupe sanguin auquel est conjugué un marqueur.

13. Kit selon la revendication 12 dans lequel la pluralité de puits comprend des puits sur lesquels sont absorbés ou auxquels sont couplés des anticorps anti-A, anti-B, anti-H et/ou anti-D.

14. Kit selon la revendication 11 qui comprend une pluralité de puits, chacun desdits puits contenant un antigène, un haptène ou un ligand spécifique d'un anticorps d'un groupe sanguin différent absorbé ou couplé sur une de ses surfaces internes et chacun desdits conjugués lyophilisés comprenant le même ou un autre antigène, haptène ou ligand spécifique dudit anticorps d'un groupe sanguin ou un second antigène spécifique dudit anticorps d'un groupe sanguin ou en compétition avec ledit premier anticorps d'un groupe sanguin vis-à-vis de la liaison audit premier antigène, haptène ou ligand auquel est conjugué un marqueur.

15. Kit selon la revendication 14, dans lequel ladite pluralité de puits inclut un ou plusieurs puits sur lesquels sont absorbées ou auxquels sont couplées des glycoprotéines synthétiques, chacune desdites glycoprotéines synthétiques comprenant une liaison oligosaccharidique à un anticorps d'un groupe sanguin couplé à une protéine.

16. Procédé d'immunoanalyse destiné à détecter la présence dans un échantillon d'une pluralité d'analytes comprenant chacun un premier membre d'un couple de liaison spécifique comprenant les étapes consistant à:

a. mettre en contact ledit échantillon avec une pluralité de substrats solides comprenant chacun une surface interne de l'un respectif d'une pluralité de puits d'une bande ou d'une boîte de microtitration et chacun desdits substrats possédant l'autre membre d'un dit couple de liaison spécifique absorbé sur ou couplé à celui-ci et un conjugué sous forme lyophilisée auquel il est associé de telle manière qu'après addition d'une solution aqueuse dudit échantillon, le conjugué lyophilisé soit reconstitué et libéré dans la solution aqueuse, et tout dit conjugué comprenant un membre de liaison susceptible de détecter la présence dudit premier membre lié à l'autre membre dudit couple de liaison spécifique sur ledit substrat et auquel est conjugué un marqueur; et

b. détecter la liaison desdits conjugués auxdits substrats solides pour révéler la présence d'un ou de plusieurs desdits premiers membres des couples de liaison spécifiques dans ledit échantillon.

17. Procédé selon la revendication 16 dans lequel chacun desdits conjugués comprend un marqueur enzymatique.

18. Procédé selon la revendication 17 dans lequel ladite enzyme est la peroxydase de raifort.

19. Procédé selon l'une des revendications 16 à 18 destiné à détecter une pluralité d'antigènes dans un échantillon, où sur chacun desdits substrats solides un anticorps spécifique d'un dit antigène est absorbé ou couplé et tout dit conjugué comprend le même ou un autre anticorps spécifique dudit antigène auquel est conjugué un marqueur.

20. Procédé selon la revendication 19 dans lequel lesdits antigènes sont des antigènes bactériens, viraux ou protozoaires.

21. Procédé selon ia revendication 20 dans lequel lesdits antigènes incluent le parvovirus canin.

22. Kit selon la revendication 19 dans lequel lesdits antigènes sont des venins de serpent.

23. Procédé selon la revendication 19 dans lequel lesdits antigènes sont des antigènes des groupes sanguins.

24. Procédé selon l'une des revendications 16 à 18 destiné à détecter une pluralité d'anticorps dans un échantillon, dans lequel chacun desdits substrats solides porte un antigène, un haptène ou un autre ligand spécifique d'undit anticorps sur lequel il est absorbé ou auquel il est couplé, et tout dit conjugué comprend le même ou un autre antigène, haptène ou ligand spécifique dudit anticorps ou un second anticorps spécifique dudit premier anticorps ou en compétition avec ledit premier anticorps vis-à-vis de la liaison au premier antigène, haptène ou ligand, auquel est conjugué un marqueur.

25. Procédé selon la revendication 24 dans lequel lesdits anticorps sont des anticorps des groupes sanguins.

26. Procédé selon la revendication 23 qui comprend la mise en contact dudit échantillon avec une pluralité de puits, chacun desdits puits contenant un anticorps spécifique d'un antigène d'un groupe sanguin différent absorbé sur ou couplé à une surface interne de celui-ci, chacun desdits conjugués lyophilisés comprenant le même ou un autre anticorps spécifique dudit antigène d'un groupe sanguin auquel est conjugué un marqueur et la détection de la liaison desdits conjugués auxdites surfaces des puits afin de révéler la présence d'un ou de plusieurs antigènes de groupes sanguins dans ledit échantillon.

27. Procédé selon la revendication 26 dans lequel ladite pluralité de puits comprend des puits sur lesquels sont absorbés ou auxquels sont couplés des anticorps anti-A, anti-B, anti-H et/ou anti-D.

28. Procédé selon la revendication 25 qui comprend la mise en contact dudit échantillon avec une pluralité de puits, chacun desdits puits contenant un antigène, un haptène ou un ligand spécifique d'un anticorps d'un groupe sanguin différent absorbé ou couplé sur une de ses surfaces internes, chacun desdits conjugués lyophilisés comprenant le même ou un autre antigène, haptène ou ligand spécifique dudit anticorps d'un groupe sanguin ou un second antigène spécifique dudit anticorps d'un groupe sanguin ou en compétition avec ledit anticorps d'un groupe sanguin vis-à-vis de la liaison audit premier antigène, haptène ou ligand auquel est conjugué un marqueur et la détection de la liaison desdits conjugués auxdites surfaces des puits afin de révéler la présence d'un ou de plusieurs desdits antigènes de groupes sanguins dans ledit échantillon.

29. Procédé selon la revendication 28, dans lequel ladite pluralité de puits inclut un ou plusieurs puits sur lesquels sont absorbées ou auxquels sont couplées des glycoprotéines synthétiques, chacune desdites glycoprotéines synthétiques comprenant une liaison oligosaccharidique à un anticorps d'un groupe sanguin couplé à une protéine.

**30.** Kit selon la revendication 1, dans lequel tout dit membre dudit couple de liaison spécifique absorbé sur ou couplé à un substrat solide est stabilisé avec de la glucosamine avant la lyophilisation dudit conjugué.

**31.** Kit selon la revendication 12, dans lequel tout dit anticorps absorbé sur ou couplé à la surface interne d'un puits est stabilisé avec de la glucosamine avant la lyophilisation dudit conjugué.

**32.** Kit selon la revendication 14, dans lequel tout dit antigène, haptène ou ligand absorbé sur ou couplé à la surface interne d'un puits est stabilisé avec de la glucosamine avant la lyophilisation dudit conjugué.

**33.** Kit selon la revendication 16, dans lequel tout dit membre dudit couple de liaison spécifique absorbé sur ou couplé à un substrat solide est stabilisé avec de la glucosamine avant la lyophilisation dudit conjugué.

**34.** Kit selon la revendication 26, dans lequel tout dit anticorps absorbé sur ou couplé à la surface interne d'un puits est stabilisé avec de la glucosamine avant la lyophilisation dudit conjugué.

**35.** Procédé selon la revendication 28, dans lequel tout dit antigène, haptène ou ligand absorbé sur ou couplé à la surface interne d'un puits est stabilisé avec de la glucosamine avant la lyophilisation dudit conjugué.

Figure 1. SENSITIVITY OF SEQUENTIAL V SIMULTANEOUS
EIA FOR DETECTION OF TIGER SNAKE VENOM

O.D. 450nm